# EUROPÄISCHE PATENTSCHRIFT

(11) **EP 1 363 128 B1**
(45) Veröffentlichungstag und Bekanntmachung des Hinweises auf die Patenterteilung: **28.06.2006**
(21) Anmeldenummer: 03010818.7
(22) Anmeldetag: 14.05.2003
(51) Int. Cl.: G01N 33/68

(54) **Diagnose von Myokardinfarkt und akutem Koronarsyndrom durch Kombination von Markern**
Diagnosis of myocardial infarction and acute coronary syndrom by combination of markers
Diagnostic de l'infarctus du myocarde et syndromes coronariens aigus par la combinaison de marqueurs

(30) Priorität: 14.05.2002 US 380413 P
(43) Veröffentlichungstag der Anmeldung: 19.11.2003
(73) Patentinhaber: F. HOFFMANN-LA ROCHE AG, 4070 Basel (CH); Roche Diagnostics GmbH, 68305 Mannheim (DE)
(72) Erfinder: Hamm, Christian, Prof., Dr., 61231 Bad Nauheim (DE); Spanuth, Eberhard, Dr., 69221 Dossenheim (DE)
(74) Vertreter: Weiss, Wolfgang

(56) Entgegenhaltungen:
- WO-A1-02/23191
- WO-A1-02/083913
- WO-A2-02/089657
- SABATINE MARC S ET AL: "Multimarker approach to risk stratification in non-ST elevation acute coronary syndromes: Simultaneous assessment of troponin I, C-reactive protein, and B-type natriuretic peptide" CIRCULATION, Bd. 105, Nr. 15, 16. April 2002 (2002-04-16), Seiten 1760-1763, XP002262207 ISSN: 0009-7322
- MAIR JOHANNES ET AL: "The impact of cardiac natriuretic peptide determination on the diagnosis and management of heart failure" CLINICAL CHEMISTRY AND LABORATORY MEDICINE, Bd. 39, Nr. 7, Juli 2001 (2001-07), Seiten 571-588, XP008024871 ISSN: 1434-6621
- HYPERTENSION, Bd. 36, Nr. 3, 2000, Seiten 355-359,
- SAKHUJA R.; JANUZZI J.L.: 'NT-proBNP - A New Test for Diagnosis, Prognosis and Management of Congestive Heart Failure' BUSINESS BRIEFING: US CARDIOLOGY 2004, Seiten 148 - 149
- MÜLLER T. ET AL: 'Long-term stability of endogenous B-type natriuretic peptide (BNP) and amino terminal proBNP (NT-proBNP) in frozen plasma samples.' CLIN CHEM LAB MED Bd. 42, Nr. 8, 2004, Seiten 942 - 944
- MÜLLER T. ET AL: 'Head-to-head comparison of the diagnostic utility of BNP and NT-proBNP in symptomatic and asymptomatic structural heart disease.' CLINICA CHIMICA ACTA Bd. 341, 2004, Seiten 41 - 48

## Beschreibung

Die vorliegende Erfindung betrifft ein Verfahren zur Diagnose von Myokardinfarkt, insbesondere ohne ST-Streckenhebung im EKG (NSTEMI) oder/und zur Risikostratifizierung des akuten Koronarsyndroms, wobei eine Bestimmung von mindestens drei Markern an einem zu untersuchenden Patienten durchgeführt wird. Weiterhin wird ein Kit zur Durchführung des Diagnoseverfahrens bereitgestellt.

Zur Diagnose von Koronarerkrankungen, wie NSTEMI und akutem Koronarsyndrom, sind eine Reihe von Markern bekannt, wie etwa Troponin T, C-reaktives Protein (CRP) und Brain-natriuretisches Peptid (BNP). Die Erhöhung der Konzentration eines dieser Marker ist mit einer Erhöhung der Wahrscheinlichkeit von ischämischen Ereignissen einschließlich Tod verbunden. Dies ist beispielsweise in den Veröffentlichungen Hamm et al. (New Engl. J. Med. 327 (1992), 146-150), Hamm et al. (New Engl. J. Med. 340 (1999), 1623-1629), Heeschen et al. (The Lancet 354 (1999), 1757-1962), Klootwijk und Hamm (The Lancet 353, Suppl. II (1999), 10-15), Wei et al. (Circulation 88 (1993), 1004-1009), De Lemos (New Engl. J. Med. 345 (2001), 1014-1021) beschrieben. Bei De Winter et al. (Cardiovasc. Res. 42 (1999), 240-245) und De Winter et al. (Clin. Chem. 46 (2000), 1597-1603) wird bereits festgestellt, dass CRP und Troponin I bzw. Troponin T zwei unabhängige Marker für die Risikostratifizierung von Patienten mit akutem Koronarsyndrom sind.

Nachteilig an bekannten Diagnoseverfahren unter Verwendung von einem oder zwei Markern ist jedoch, dass eine vollständige Erfassung von Risikopatienten nicht gelingt. Eine der Erfindung zugrunde liegende Aufgabe bestand somit darin, ein Verfahren zur Diagnose von Myokardinfarkt oder/und zur Risikostratifizierung des akuten Koronarsyndroms zu entwickeln, das eine verbesserte Erfassung von Risikopatienten ermöglicht.

Diese Aufgabe wird dadurch gelöst, dass eine Bestimmung von mindestens drei Markern an einer oder mehreren Blut- oder Serumproben aus einem zu untersuchenden Patienten durchgeführt wird, wobei jeweils mindestens NT-ProBNP, mindestens ein ischämischer Marker und mindestens ein inflammatorischer Marker bestimmt wird.

Überraschenderweise wurde festgestellt, dass NT-Pro BNP ischämische Marker und inflammatorische Marker Gruppen von jeweils unabhängigen Risikoindikatoren darstellen, so dass sich durch die erfindungsgemäße Kombination dieser Markergruppen eine additiv erhöhte Sensitivität und Spezifität und damit auch höhere prädiktive Werte und eine höhere diagnostische Effizienz bei der Identifizierung von Patienten mit erhöhtem Risiko oder/und ungünstiger Prognose ergeben. Dies führt wiederum dazu, dass bei den getesteten Patienten eine verbesserte Indikationsstellung für geeignete therapeutische Maßnahmen vorgenommen werden kann.

Ein sich daraus ergebender Vorteil ist, dass bei Vorliegen kardialer Erkrankungen, insbesondere Myokardinfarkt ohne ST-Streckenhebung im EKG (NSTEMI), eine höhere Anzahl von Patienten mit erhöhtem Risiko oder/und ungünstiger Prognose identifiziert und adäquat behandelt werden kann als beim gegenwärtigen diagnostischen Vorgehen, welche eines Bestimmung einzelner Marker erfasst. Durch die erfindungsgemäße Kombination von drei unterschiedlichen Markern bei der Diagnosestellung kann die Häufigkeit von Todesfällen und anderer kardialer Komplikationen weiter reduziert werden.

Das erfindungsgemäße Verfahren umfasst die Bestimmung von mindestens drei Markern, wobei mindestens NT-ProBNP, mindestens ein ischämischer Marker und mindestens ein inflammatorischer Marker bestimmt wird.

Als ischämischer Marker können beispielsweise Troponin T oder Troponin I bestimmt werden. Besonders bevorzugt wird Troponin T als ischämischer Marker bestimmt.

Der inflammatorische Marker kann beispielsweise ausgewählt werden aus C-reaktivem Protein (CRP), Interleukinen, insbesondere IL-6, und Adhäsionsmolekülen, wie VCAM und ICAM. Vorzugsweise wird als inflammatorischer Marker CRP oder IL-6 bestimmt.

Die erfindungsgemäße Kombinationsbestimmung wird vorzugsweise so durchgeführt, dass an einer oder mehreren Blut- oder Serumproben aus einem zu untersuchenden Patienten parallele Bestimmungen der Marker durchgeführt werden. Vorzugsweise werden eine oder mehrere aus dem Patienten stammende Blut- oder Serumproben, in einem oder mehreren Tests gleichzeitig oder unmittelbar hintereinander untersucht. Besonders bevorzugt werden die Bestimmungen an einer einzigen Patientenprobe durchgeführt.

Die kombinierte Bestimmung der Marker kann grundsätzlich nach beliebigen bekannten Methoden mit marktgängigen kommerziellen Tests durchgeführt werden. Zur Bestimmung können beispielsweise Analyseautomaten eingesetzt werden. Alternativ können auch Schnelltests, beispielsweise für die Anwendung in der Notaufnahme, in der Station oder Intensivstation, in der Ambulanz oder Arztpraxis oder als Patientenselbsttest eingesetzt werden.

Die Bestimmung der Marker erfolgt üblicherweise durch einen Immunoassay unter Verwendung von gegen den Marker gerichteten Antikörpern. Zum Nachweis von C-reaktivem Protein als inflammatorischer Marker wird beispielsweise auf Liuzzo et al. (N. Engl. J. Med. 331 (1994), 417-424), Kuller et al. (Am. J. Epidem. 144 (1996), 537-547), Price et al. (J. Immunol. Methods. 99 (1987), 205-211) oder Eda et al. (J. Clin. Lab. Anal. 12 (1998), 137-144) verwiesen. Ein besonders bevorzugter Test zum Nachweis von C-reaktivem Protein ist ein immunturbidimetrischer Test, beispielsweise der Tina-Quant® -Test von Roche Diagnostics GmbH, Mannheim.

Der Nachweis von NT-ProBNP als neurohomonaler Marker ist beispielsweise bei Richards et al. (Circulation 97 (1998), 1921-1929), Struthers (Eur. Heart J. 20 (1999), 1374-1375), Hunt et al. (Clin. Endocrinol. 47 (1997), 287-296), Talwar et al. (Eur. Heart J. 20 (1999), 1736-1744), Darbar et al. (Am. J. Cardiol. 78 (1996), 284-287) sowie in EP-A-0 648 228 und WO 00/45176 beschrieben. Ein besonders bevorzugter Test ist ein Elektrochemilumineszenz-lmmunoassay, z.B. das Testformat "ECLIA" von Roche Diagnostics GmbH, Mannheim.

Bezüglich der Bestimmung von Troponin T als Beispiel für einen ischämischen Marker wird auf Katus et al. (Mol. Cell. Cardiol. 21 (1989), 1349-1353), Hamm et al. (N. Engl. J. Med. 327 (1992), 146-150), Ohmann et al. (N. Engl. J. Med. 335 (1996), 13333-1334), Christenson et al. (Clin. Chem. 44 (1998), 494-501) und zahlreiche andere Publikationen sowie auf EP-A-0 394 819 verwiesen. Besonders bevorzugte Tests zum Nachweis von Troponin T sind Elektrochemilumineszenz-lmmunoassays, z.B. die Testformate Elecsys® Troponin T- und Elecsys® Troponin T STAT von Roche Diagnostics GmbH, Mannheim.

Noch ein weiterer Aspekt der Erfindung ist ein Reagenzienkit zur Diagnose von akutem Koronarsyndrom, der Nachweisreagenzien zur Bestimmung von mindestens drei Markern enthält, wobei jeweils mindestens ein Nachweisreagenz für NT-ProBNP, jeweils mindestens ein Nachweisreagenz für einen ischämischen Marker und jeweils mindestens ein Nachweisreagenz für einen inflammatorischen Marker vorliegt.

Der Reagenzienkit ist vorzugsweise so ausgestaltet, dass er sich zur Durchführung von parallelen Bestimmungen der Marker und insbesondere zur Durchführung der Bestimmungen an einer einzigen Patientenprobe eignet. Hierfür ist es zweckmäßig, Nachweisreagenzien zu verwenden, die eine Bestimmung aller drei Marker durch ein einziges Testformat ermöglichen, beispielsweise einen Enzymuntest, einen Elektrochemilumineszenztest, einen turbidimetrischen Test oder einen Schnelltest auf einem Teststreifen.

Der Reagenzienkit kann zur Identifizierung von Patienten mit akutem Koronarsyndrom eingesetzt werden, die ein erhöhtes Risiko oder/und eine ungünstige Prognose aufweisen. Der Reagenzienkit kann so ausgestaltet sein, dass er sich zur Durchführung der Bestimmungen an einem Analyseautomaten oder Schnelltest eignet.

Weiterhin soll die Erfindung durch Beispiele und Figuren erläutert werden.

### Beispiele

### Beispiel 1

Es wurden Untersuchungen der drei Marker Troponin T, NT-ProBNP und CRP an 500 Patienten mit akutem Koronarsyndrom (NSTEMI) durchgeführt. Zur Bestimmung von Troponin T wurde der Troponin T STAT-Test von Roche Diagnostics GmbH, Mannheim, zur Bestimmung von CRP, der Tina-Quant® -CRP-Test von Roche Diagnostics GmbH, Mannheim und zur Bestimmung von NT-ProBNP der ECLIA-Test von Roche Diagnostics, Mannheim, jeweils nach Angaben des Herstellers verwendet.

Alle drei Parameter differenzieren zwischen Patienten mit ungünstiger 30 Tage-Prognose hinsichtlich Tod und Myokardinfarkt (Ml) und Patienten mit ereignisfreiem 30 Tagesverlauf.

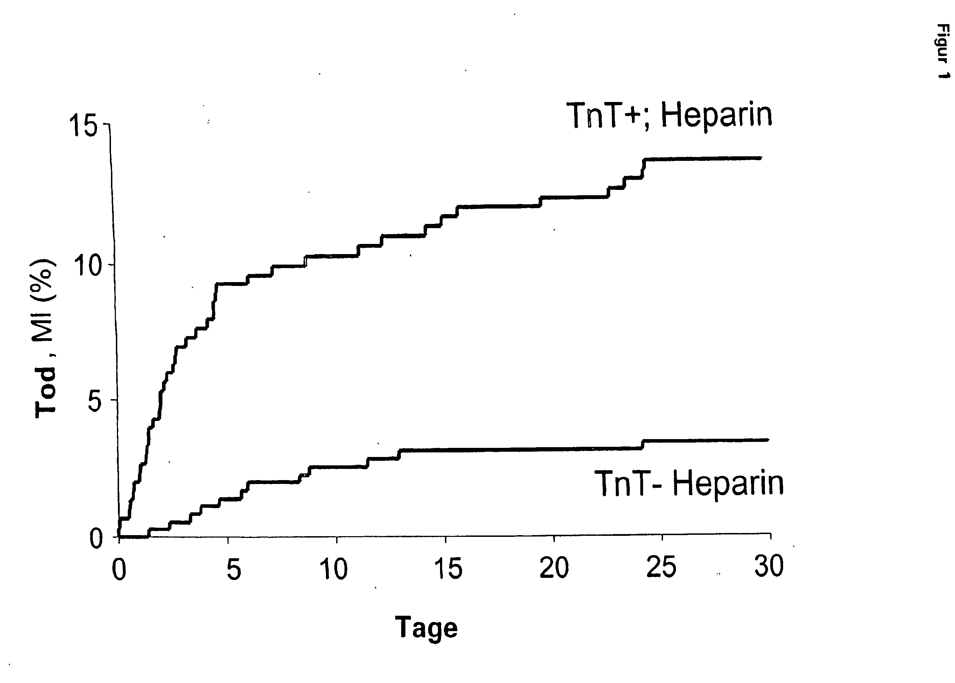

**Figur 1** zeigt den Verlauf für Troponin T (TnT).

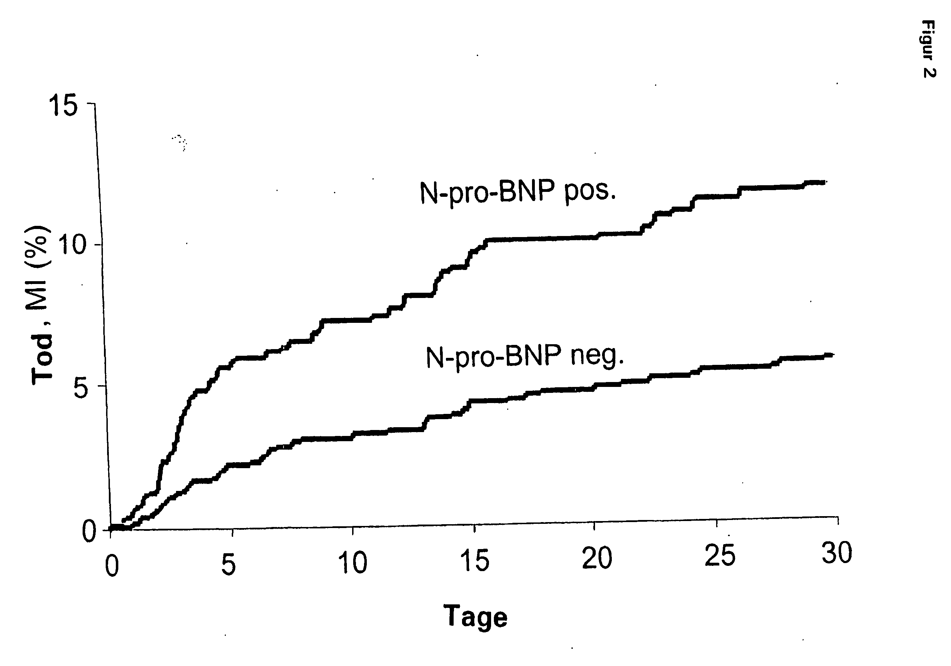

**Figur 2** zeigt den Verlauf für NT-ProBNP.

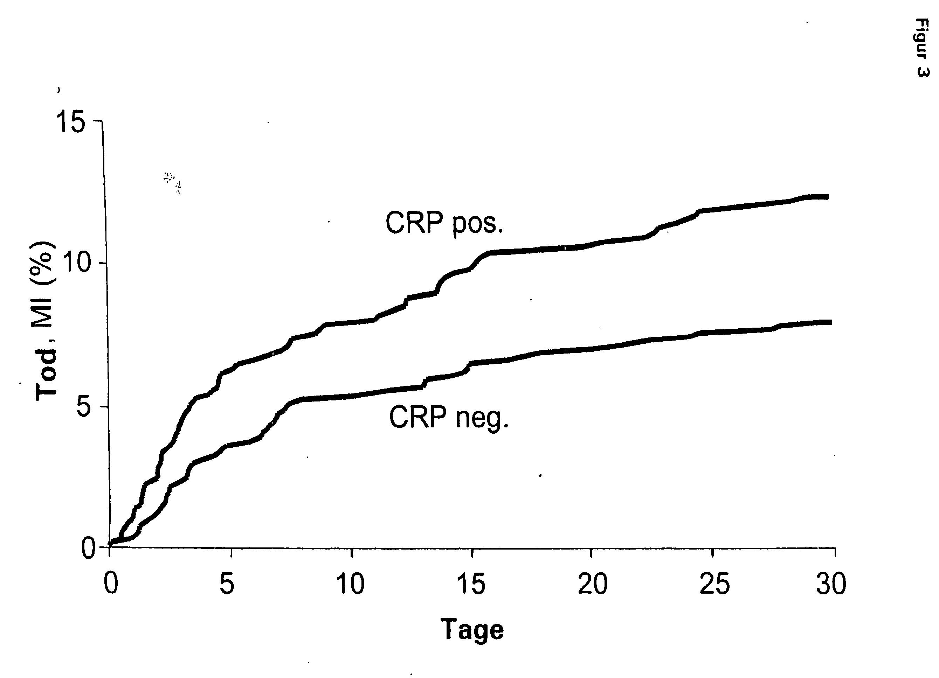

**Figur 3** zeigt den Verlauf für CRP.

Darüber hinaus wurde durch eine multivariante Analyse gefunden, dass die drei Parameter voneinander unabhängig sind.

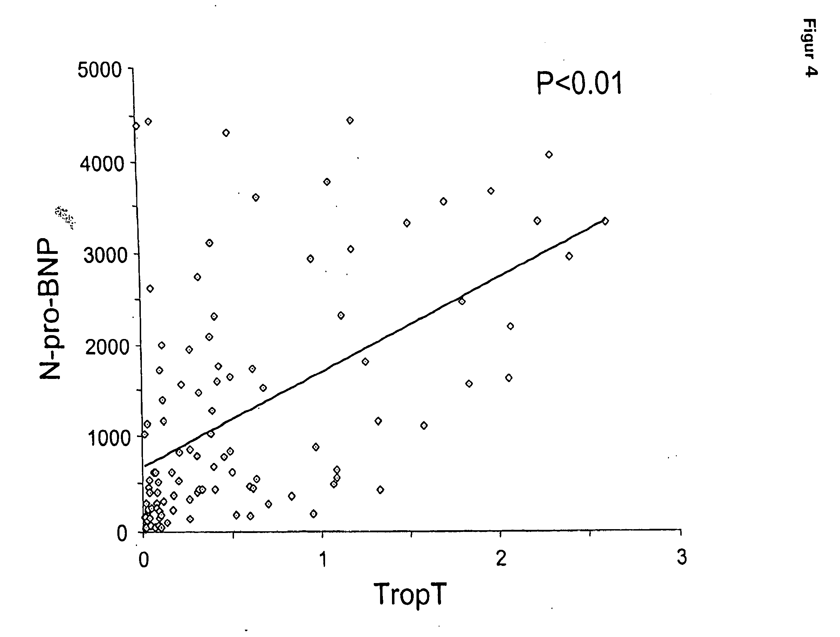

**Figur 4** zeigt die Korrelation zwischen dem NT-ProBNP-Wert und dem Troponin T-Wert. Es ist zu erkennen, dass einige der untersuchten Patienten nur bezüglich eines der Parameter erhöhte Werte aufweisen.

### Beispiel 2

Es wurden Untersuchungen der drei Marker Troponin T, NT-ProBNP und CRP an 1848 Patienten durchgeführt. Zur Bestimmung aller drei Parameter wurde der jeweilige ELISA-Test von Roche verwendet. Es konnten die Ergebnisse von Beispiel 1 bestätigt werden. Für den Troponin T-Test (Nachweisgrenze 0,01 pg/l) wurde ein Cut-off-Wert (Unterscheidung zwischen positiv und negativ) von 0,1 pg/l verwendet. Für den NT-ProBNP-Test (Nachweisgrenze 5 ng/l) wurde ein Cut-off-Wert von 400 ng/l verwendet. für den CRP-Test (Nachweisgrenze 0,5 mg/l) wurde ein Cut-off-Wert von 15,0 mg/l verwendet. Der NT-ProBNP-Wert ist insbesondere für eine kurzfristige Prognosestellung, z.B. für den Verlauf der nächsten 72 h, von großer Bedeutung.

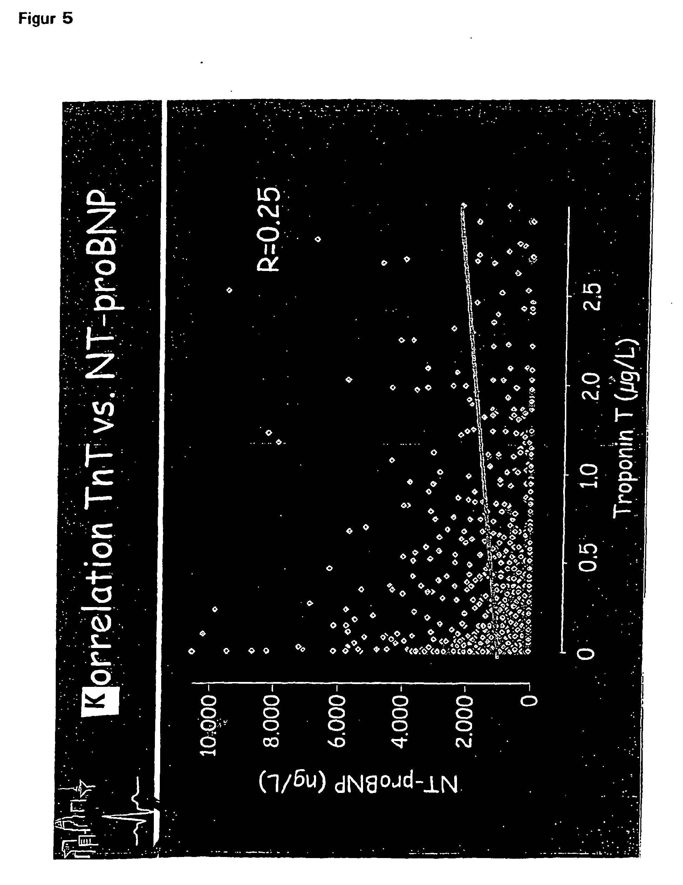

**Figur 5** zeigt die Korrelation zwischen dem NT-ProBNP-Wert und dem Troponin T-Wert.

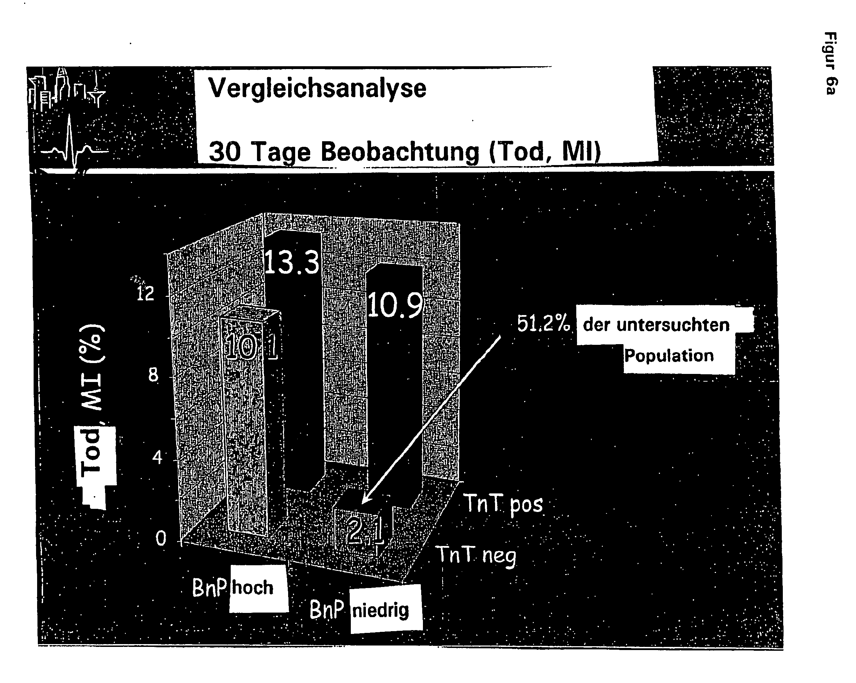

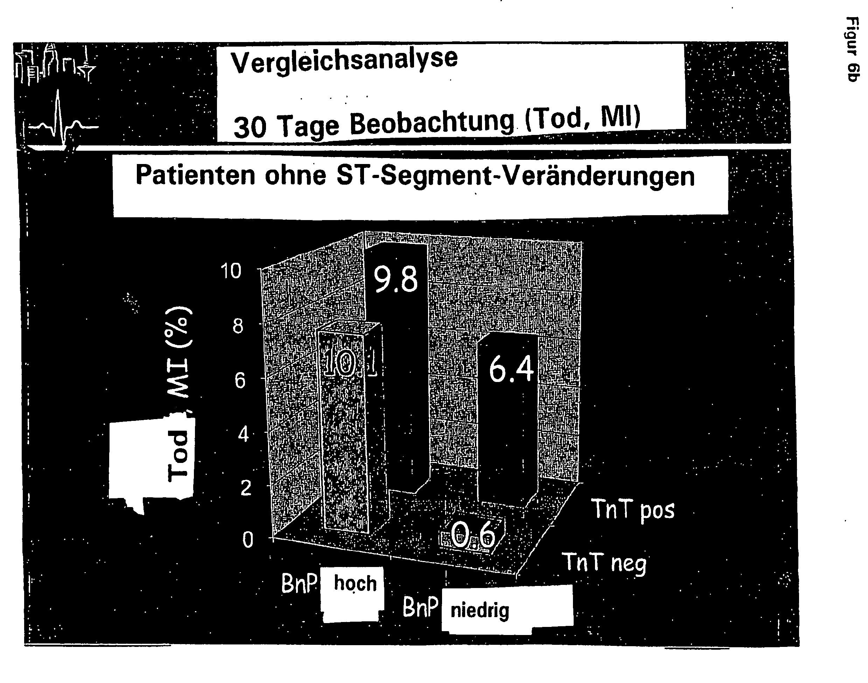

**Figur 6** zeigt das Ergebnis einer 30-Tagesverlaufskontrolle für den Anteil aller Patienten (a) und der NSTEMI-Patienten (b) mit Tod bzw. Myokardinfarkt (Ml) hinsichtlich der Parameter NT-ProBNP und Troponin T sowie deren Kombination.

### Verwendete Abkürzungen:

- NSTEMI:: Non ST-segment elevation myocardial infarction (akutes Koronarsyndrom)
- VCAM:: vc adhesion molecule (vc Adhäsionsmolekül)
- ICAM:: ic adhesion molecule (ic Adhäsionsmolekül)
- ECLIA:: electrochemiluminescence immuno assay (Elektrochemilumineszenz-Immunoassay)

## Patentansprüche

1. Verfahren zur Diagnose von Myokardinfarkt oder/und zur Risikostratifizierung des akuten Koronarsyndroms,
**dadurch gekennzeichnet,**
**dass** eine Bestimmung von mindestens drei Markern an einer oder mehreren Blut- oder Serumproben aus einem zu untersuchenden Patienten durchgeführt wird, wobei jeweils mindestens ein ischämischer Marker, mindestens ein inflammatorischer Marker und NT-ProBNP als neurohormonaler Marker bestimmt wird.

2. Verfahren nach Anspruch 1,
**dadurch gekennzeichnet,**
**dass** der ischämische Marker aus Troponin T und Troponin I ausgewählt wird.

3. Verfahren nach Anspruch 2,
**dadurch gekennzeichnet,**
**dass** Troponin T als ischämischer Marker bestimmt wird.

4. Verfahren nach Anspruch 1,
**dadurch gekennzeichnet,**
**dass** der inflammatorische Marker ausgewählt wird aus C-reaktivem Protein (CRP), Interleukinen, wie etwa IL-6, und Adhäsionsmolekülen, wie VCAM oder ICAM.

5. Verfahren nach Anspruch 4,
**dadurch gekennzeichnet,**
**dass** CRP oder/und IL-6 als inflammatorischer Marker bestimmt wird.

6. Verfahren nach einem der Ansprüche 1 bis 5 zur Identifizierung von Patienten mit erhöhtem Risiko oder/und einer ungünstigen Prognose.

7. Verfahren nach einem der Ansprüche 1 bis 6,
**dadurch gekennzeichnet,**
**dass** parallele Bestimmungen der Marker durchgeführt werden.

8. Verfahren nach einem der Ansprüche 1 bis 7,
**dadurch gekennzeichnet,**
**dass** die Bestimmungen an einer einzigen Patientenprobe durchgeführt werden.

9. Verfahren nach einem der Ansprüche 1 bis 8,
**dadurch gekennzeichnet,**
**dass** die Bestimmungen an einem Analyseautomaten durchgeführt werden.

10. Verfahren nach einem der Ansprüche 1 bis 8,
**dadurch gekennzeichnet,**
**dass** die Bestimmungen als Schnelltest durchgeführt werden.

11. Reagenzienkit zur Diagnose von Myokardinfarkt oder/und zur Risikostratifizierung des akuten Koronarsyndroms,
**dadurch gekennzeichnet,**
**dass** er Nachweisreagenzien zur Bestimmung von mindestens drei Markern enthält, wobei jeweils mindestens ein Nachweisreagenz für den neurohormonalen Marker NT-ProBNP, jeweils mindestens ein Nachweisreagenz für einen ischämischen Marker und jeweils mindestens ein Nachweisreagenz für einen inflammatorischen Marker vorliegt.

12. Verwendung eines Reagenzienkits nach Anspruch 11 zur Durchführung von parallelen Bestimmungen der Marker in einer oder mehreren Blut- oder Serumproben aus einem zu unter suchenden Patienten.

13. Verwendun eines Reagenzienkits nach Anspruch 11 oder 12 zur Durchführung der Bestimmungen in einer einzigen Blut oder Serumprobe aus einem zu untersuchenden Patienten.

14. Verwendung eines Reagenzienkits nach einem der Ansprüche 11 bis 13 zur Durchführung der Bestimmungen an einem Analyseautomaten in einer oder mehreren Blut- oder Serumproben aus einem zu untersuchenden Patienten.

15. Verwendung eines Reagenzienkits nach einem der Ansprüche 11 bis 14 zur Durchführung der Bestimmungen als Schnelltest in einer oder mehreren Blut- oder Serumproben aus einem zu untersuchenden Patienten..

16. Verwendung eines Reagenzienkits nach einem der Ansprüche 11 bis 15 zur Identifizierung von Patienten mit akutem Koronarsyndrom, die ein erhöhtes Risiko oder/und eine ungünstige Prognose aufweisen in einer oder mehreren Blut- oder Serumproben aus einem zu untersuchenden Patienten.

## Claims

1. Method for the diagnosis of myocardial infarction or/and for the risk stratification of acute coronary syndrome,
**characterized in that**
at least three markers are determined in one or more blood or serum samples from a patient to be examined, where at least one ischemic marker, at least one inflammatory marker and NT-proBNP as a neurohormonal marker are determined in each case.

2. Method according to claim 1,
**characterized in that**
the ischemic marker is selected from troponin T and troponin I.

3. Method according to claim 2,
**characterized in that**
troponin T is determined as the ischemic marker.

4. Method according to claim 1,
**characterized in that**
the inflammatory marker is selected from C-reactive protein (CRP), interleukins such as IL-6 and adhesion molecules such as VCAM or ICAM.

5. Method according to claim 4,
**characterized in that**
CRP or/and IL-6 is determined as the inflammatory marker.

6. Method according to one of the claims 1 to 5 for identifying patients with an elevated risk or/and an unfavourable prognosis.

7. Method according to one of the claims 1 to 6,
**characterized in that**
the markers are determined concurrently.

8. Method according to one of the claims 1 to 7,
**characterized in that**
the determinations are carried out on a single patient sample.

9. Method according to one of the claims 1 to 8,
**characterized in that**
the determinations are carried out on an automated analyzer.

10. Method according to one of the claims 1 to 8,
**characterized in that**
the determinations are carried out as a rapid test.

11. Reagent kit for diagnosing myocardial infarction or/and for the risk stratification of the acute coronary syndrome,
**characterized in that**
it contains detection reagents for determining at least three markers where at least one detection reagent for the neurohormonal marker NT-proBNP is present in each case, at least one detection reagent for an ischemic marker is present in each case and at least one detection reagent for an inflammatory marker is present in each case.

12. Use of a reagent kit according to claim 11 to perform concurrent determinations of the markers in one or more blood or serum samples from a patient to be examined.

13. Use of a reagent kit according to claim 11 or 12 to perform determinations in a single blood or serum sample from a patient to be examined.

14. Use of a reagent kit according to one of the claims 11 to 13 to perform determinations in one or more blood or serum samples from a patient to be examined on an automated analyzer.

15. Use of a reagent kit according to one of the claims 11 to 14 to perform determinations as a rapid test in one or more blood or serum samples from a patient to be examined.

16. Use of a reagent kit according to one of the claims 11 to 15 to identify patients with acute coronary syndrome who have an elevated risk or/and an unfavourable prognosis in one or more blood or serum samples from a patient to be examined.

## Revendications

1. Procédé de diagnostic de l'infarctus du myocarde et/ou de stratification du risque du syndrome coronarien aigu, **caractérisé en ce que** l'on effectue une détermination d'au moins trois marqueurs sur un ou plusieurs échantillons de sang ou de sérum d'un patient à examiner, en déterminant à chaque fois au moins un marqueur ischémique, au moins un marqueur inflammatoire et le NT-ProBNP comme marqueur neurohormonal.

2. Procédé selon la revendication 1, **caractérisé en ce que** le marqueur ischémique est choisi parmi la troponine T et la troponine I.

3. Procédé selon la revendication 2, **caractérisé en ce que** l'on détermine la troponine T comme marqueur ischémique.

4. Procédé selon la revendication 1, **caractérisé en ce que** le marqueur inflammatoire est choisi parmi la protéine réactive C (CRP), des interleukines comme, par exemple, l'IL-6, et des molécules d'adhésion, comme la VCAM ou l'ICAM.

5. Procédé selon la revendication 4, **caractérisé en ce que** l'on détermine la CRP et/ou l'IL-6 comme marqueur inflammatoire.

6. Procédé selon l'une des revendications 1 à 5 pour l'identification de patients ayant un risque élevé et/ou un pronostic défavorable.

7. Procédé selon l'une des revendications 1 à 6, **caractérisé en ce que** l'on effectue des déterminations parallèles des marqueurs.

8. Procédé selon l'une des revendications 1 à 7, **caractérisé en ce que** l'on effectue les déterminations sur un seul échantillon du patient.

9. Procédé selon l'une des revendications 1 à 8, **caractérisé en ce que** l'on effectue les déterminations dans un appareil d'analyse automatique.

10. Procédé selon l'une des revendications 1 à 8, **caractérisé en ce que** l'on effectue les déterminations sous forme de test rapide.

11. Kit de réactifs pour le diagnostic de l'infarctus du myocarde et/ou la stratification du risque du syndrome coronarien aigu, **caractérisé en ce qu'**il contient des réactifs de détection pour la détermination d'au moins trois marqueurs, avec à chaque fois au moins un réactif de détection du marqueur neurohormonal NT-ProBNP, à chaque fois au moins un réactif de détection d'un marqueur ischémique et à chaque fois au moins un réactif de détection d'un marqueur inflammatoire.

12. Utilisation d'un kit de réactifs selon la revendication 11 pour la mise en oeuvre de déterminations parallèles des marqueurs dans un ou plusieurs échantillons de sang ou de sérum d'un patient à examiner.

13. Utilisation d'un kit de réactifs selon la revendication 11 ou 12 pour la mise en oeuvre des déterminations sur un seul échantillon de sang ou de sérum d'un patient à examiner.

14. Utilisation d'un kit de réactifs selon l'une des revendications 11 à 13 pour la mise en oeuvre des déterminations dans un appareil d'analyse automatique dans un ou plusieurs échantillons de sang ou de sérum d'un patient à examiner.

15. Utilisation d'un kit de réactifs selon l'une des revendications 11 à 14 pour la mise en oeuvre des déterminations sous forme de test rapide dans un ou plusieurs échantillons de sang ou de sérum d'un patient à examiner.

16. Utilisation d'un kit de réactifs selon l'une des revendications 11 à 15 pour l'identification de patients ayant un syndrome coronarien aigu, qui présentent un risque élevé et/ou un pronostic défavorable, dans un ou plusieurs échantillons de sang ou de sérum d'un patient à examiner.
